# EUROPEAN PATENT APPLICATION

(11) **EP 4 674 393 A1**
(43) Date of publication of application: **07.01.2026**
(21) Application number: 24186591.4
(22) Date of filing: 04.07.2024
(51) Int. Cl.: A61F 9/00, A61F 9/007

(54) **NEEDLE AND NEEDLE SYSTEM FOR SUBRETINAL INJECTION**

(71) Applicant: Carl Zeiss Meditec AG, 07745 Jena (DE)
(72) Inventor: MEENINK, Hildebert Christiaan Matthijs, 5612 AP Eindhoven (NL)
(74) Representative: DeltaPatents B.V.

(57) **Abstract**

A needle is provided for subretinal injection. The needle may comprise a concentric assembly 100 forming a lumen 140 for a substance to be injected below a retinal surface layer, the concentric assembly comprising an outer tube 130, an intermediate tube 120, and an inner tube 110 comprising a tip 116 for piercing the retinal surface layer, wherein the tip is at a distal end of the inner tube, wherein the inner tube extends axially from a distal end of the intermediate tube, wherein the inner tube is at least partially translucent to optical coherence tomography (OCT), wherein the tip is beveled, wherein the inner tube has an inner tube outer diameter of 120µm or less at an onset of the bevel. Furthermore, a needle system is provided which comprises the needle and a flexible tubing to supply the substance to the needle.

## Description

### TECHNICAL FIELD

The presently disclosed subject matter relates to a needle for subretinal injection, to a needle system comprising the needle and a flexible tubing, and a use of the needle and the needle system for performing a subretinal injection.

### BACKGROUND

Surgical instruments used in posterior ophthalmic surgery are typically minimally invasive and microsurgical in nature. Such instruments may be used to operate on the retina. The total thickness of the retina may range from 150 to 300 µm, with an average of approximately 200 µm. Therefore, high precision may be required in the surgical instruments themselves and in the handling of these surgical instruments.

Needles, which may be a general term for surgical instruments designed to penetrate tissues and deliver substances, may be used for subretinal injections. Such needles may also be referred to as subretinal injection (SRI) needles or simply as SRI needles. For example, to treat existing and novel diseases, treatment may involve using an SRI needle to inject a small amount (e.g., 10 µL) of a therapeutic agent within the retina. If the needle is not placed correctly when the therapeutic agent is injected, the therapeutic agent may flow back into the vitreous cavity where it may cause an inflammatory reaction, or when the needle is inserted below the retina, the Bruch's membrane may be damaged, compromising an immunological barrier of the human body. Therefore, precise placement of the needle may be of utmost importance and may require a high degree of control. For example, a high degree of control may be needed in penetrating a retinal surface layer with the tip of the needle, positioning the tip of the needle at a suitable depth below the retinal surface layer, and if the position is satisfactory, injecting the therapeutic agent in a controlled manner.

Some of the challenges in achieving such precision may be addressed by surgical robotic systems. These systems may operate a surgical instrument, such as a needle with enhanced precision, improved dexterity, steadier instrument manipulation, and the ability to scale movements, compared to a fully manually held needle.

The needle design may also play a pivotal role in the success of subretinal injections. The needle design may ensure that a needle can penetrate the retina without causing excessive damage and deliver the therapeutic agent accurately to the intended subretinal space. A suitable needle design may be valuable irrespective of whether the needle is held by a surgical robotic system or by a surgeon.

CN115137553A describes a subretinal fluid discharge device. The device comprises a needle telescopically arranged in an accommodating cavity. The needle, which may be a 41G micro-fine needle, may be extended or retracted.

Further improvements may be desirable in the design of a needle for subretinal injection. Moreover, further improvements may be desirable in the needle system which connects the needle to a substance supply, e.g., to a syringe.

### SUMMARY

A first aspect of the presently disclosed subject matter provides a needle for subretinal injection, the needle comprising a concentric assembly forming a lumen for a substance to be injected below a retinal surface layer, the concentric assembly comprising:
- an outer tube comprising an outer tube outer diameter; and
- an inner tube comprising a tip for piercing the retinal surface layer,
wherein the tip is at a distal end of the inner tube, wherein the inner tube extends axially from a distal end of the outer tube, wherein the inner tube is at least partially translucent to optical coherence tomography (OCT), wherein the tip is beveled, wherein the inner tube has an inner tube outer diameter of 120µm or less at an onset of the bevel; and
wherein the concentric assembly is arranged to circumferentially taper-down along a longitudinal direction towards the tip from the outer tube outer diameter, via at least one intermediate outer diameter, to the inner tube outer diameter.

The above-described aspect of the invention may provide a needle, which may refer to a surgical instrument designed to penetrate tissues and deliver substances. The needle may be designed for subretinal injection of a substance, such as a therapeutic agent. For that purpose, the needle may comprise an internal lumen through which the substance may be delivered. To subretinally inject the substance, the needle may comprise a tip. The tip may be arranged at a distal end of the needle and may comprise an opening through which the substance may be ejected from the needle. The tip may additionally enable the needle to pierce the retinal surface layer.

The presently disclosed subject matter may provide a needle which comprises a concentric assembly of at least two tubes which form the lumen for the substance to be injected. The concentric assembly may comprise an inner tube and an outer tube. In a radial plane, which may be perpendicular to a longitudinal axis of both tubes, the inner tube may be concentrically aligned within the outer tube. In a longitudinal plane, which may be parallel to a length of both tubes, the inner tube may extend axially beyond the distal end of the outer tube, protruding outward. The inner tube may comprise the tip of the needle. By protruding outward from the outer tube, the tip and immediately adjacent shaft section of the inner tube may pierce the retinal surface layer and enter the retina, while the outer tube remains outside of the retina.

The inner tube may be manufactured from a material which is at least partially translucent to optical coherence tomography (OCT). For example, such partial translucency to OCT may be characterized by the material passing at least a measurable amount of the OCT wavelengths, typically in the range of 800 nm to 1300 nm. As such, while the material may not need to be fully transparent to OCT, the material is not completely opaque to OCT and rather may have an intermediate level of translucency. For example, the material may be partially translucent to visible light and thereby partially translucent to OCT. Moreover, the partial translucency may be a translucency to such a degree that the material may be considered to be partially transparent to OCT. By the inner tube being at least partially translucent, the tip and immediately adjacent shaft section of the inner tube are partially translucent as well. This may be advantageous since subretinal injections increasingly utilize OCT as a visualization technique to obtain feedback on where and how to pierce the tissue with the needle, as well as to determine the appropriate insertion depth of the needle.

Since the retina is extremely thin, precise depth placement may be crucial. The inventors have determined that partial translucency may allow for better visualization. Namely, a partially translucent inner tube may allow the cross-section of the needle lumen to be seen in OCT imaging, which may be significant because the substance is ejected from the exit aperture of the lumen at the needle tip, and it may be critical that the exit aperture is positioned below the retinal surface layer before the substance is ejected to reduce the chance of the injected substance flowing back into the vitreous cavity and above the retinal pigment epithelium to avoid complications such as subretinal hemorrhages, an inflammation reaction, etc. In other words, it may be critical that the needle is positioned with its tip, and thereby with the exit aperture of the lumen, at a suitable injection depth before starting injection. The transparency may facilitate the placement of the exit aperture of the lumen within the retina at a suitable injection depth. In contrast, non-translucent materials, such as metals, may reflect OCT light, making it difficult to accurately assess the positioning of the exit aperture relative to the retina.

The inventors have further determined that materials that are at least partially translucent to OCT light are often flexible, which may result in bending or buckling when attempting to penetrate the retinal surface layer. To address this potential deficiency, the tip of the inner tube may be beveled and the inner tube may have an outer diameter of 120µm or less at an onset of the bevel. This tip design may facilitate cutting through the retinal surface layer and thereby improve penetration performance and reduce damage to the retinal surface layer. To further address the potential bending or buckling, the concentric assembly may be configured to circumferentially taper along a longitudinal direction towards the tip, starting from the outer diameter of the outer tube, through at least one intermediate outer diameter, to the outer diameter of the inner tube. In other words, the concentric assembly may comprise an intermediate needle section with an intermediate outer diameter. This tapering may for example be stepwise or gradual. The tapered design of the concentric assembly may be advantageous since the outer tube may ensure axial stability of the inner tube and may thereby reduce the risk of bending or buckling of the inner tube. However, if the inner tube protrudes insufficiently from the outer tube, visibility may be impaired, especially when viewing the injection site along the axial direction, for example from a microscope. Conversely, if the inner tube protrudes too far from the outer tube, the inner tube may lack adequate axial stability. The intermediate diameter may mitigate these issues by obstructing visibility less compared to a needle section with the outer diameter of the outer tube, while still providing greater axial stability compared to a needle section with the outer diameter of the inner tube.

Optionally, the inner tube outer diameter is 110µm or less, preferably 100µm or less, more preferably 80µm ± 10µm. By comprising a comparatively small outer diameter, the exit aperture of the lumen at the tip of the needle may be more easily positioned to entirely remain within the retina and below the retinal surface layer, thereby reducing the chance of the substance flowing back into the vitreous cavity.

Optionally, the bevel of the tip is beveled at an angle of 45° ± 10°, preferably 45° ± 5°, more preferably 45° ± 2.5°. A bevel angle of substantially 45° has been found to provide a sharp tip which may facilitate the penetration through the retinal surface layer, while at the same time keeping the diameter of the exit aperture of the lumen relatively small, thereby further facilitating the positioning of the tip of the needle to entirely remain within the retina and below the retinal surface layer.

Optionally, the inner tube comprises or is made out of a translucent plastic, preferably a translucent thermoplastic, more preferably a translucent polymer such as polyimide. Translucent plastics such as polyimide are well-suited as materials for the inner tube.

Optionally, the concentric assembly further comprises an intermediate tube concentrically arranged between the inner tube and the outer tube, the intermediate tube comprising the intermediate outer diameter, wherein the intermediate tube extends axially from the distal end of the outer tube and the inner tube extends axially from a distal end of the intermediate tube. The tapered design of the concentric assembly may at least in part be established by an intermediate tube. In the aforementioned radial plane, the intermediate tube may be arranged concentrically between the outer tube and the inner tube, and in the aforementioned longitudinal plane, the intermediate tube may extend axially beyond the distal end of the outer tube, protruding outwardly, while the inner tube may extend axially beyond the distal end of the intermediate tube, also protruding outwardly. The longitudinal profile of the concentric assembly may thus exhibit a stepwise taper, providing the aforementioned advantages of less obstruction of visibility compared to an intermediate needle section with the outer diameter of the outer tube, and greater axial stability compared to an intermediate needle section with the outer diameter of the inner tube.

Optionally, the inner tube extends axially from the distal end of the intermediate tube by a length of 1mm or less, preferably 750µm or less, more preferably 500µm ± 100µm. The outward protrusion of the inner tube from the intermediate tube by any of these lengths has been found to provide sufficient axial stability while still allowing sufficient visibility of the insertion site.

Optionally, the intermediate tube extends axially from the distal end of the outer tube by a length of 1mm to 3mm, preferably 2mm ± 500µm, more preferably 2mm ± 250µm. The outward protrusion of the intermediate tube from the outer tube by any of these lengths has been found to allow sufficient observability of the insertion site.

Optionally, the inner tube outer diameter is selected to substantially match an intermediate inner diameter of the intermediate tube; and/or an intermediate tube outer diameter of the intermediate tube is selected to substantially match an outer tube inner diameter of the outer tube. The concentric assembly may thus comprise three nested tubes, each with diameters closely matched to ensure a close tolerance. By providing such a nested assembly, the use of additional connecting structures between the tubes may be avoided, and the potential for air cavities, which may otherwise manifest themselves in such connecting structures, may be reduced. Air cavities may generally be undesirable in a needle since they may lead to inaccuracies in dosing.

Optionally, the intermediate tube is at least partially translucent to OCT, for example by comprising or being made out of out of a translucent plastic, preferably a translucent thermoplastic, more preferably a translucent polymer such as polyimide. By also providing a partially translucent intermediate tube, a better visualization of the needle at the (potential) insertion site may be obtained. Namely, while the intermediate tube may not be in direct contact with the retinal surface layer, the intermediate tube may nevertheless be positioned proximate to the insertion site, where an intermediate tube manufactured from a non-translucent material would reflect OCT light, potentially creating artifacts or glare that may obscure the visualization of the injection site.

Optionally, the inner tube is attached to an inside of the intermediate tube, and/or the intermediate tube is attached to an inside of the outer tube. By directly attaching a pair of tubes to each other, for example using an adhesive such as glue, additional connecting structures between the tubes may be avoided and manufacturing may be facilitated.

Optionally, the intermediate tube has an intermediate tube outer diameter of between 140µm and 180µm, preferably 160µm ± 10µm.

Optionally, the inner tube is circumferentially tapered-down along a longitudinal direction from or via the intermediate outer diameter to the inner tube outer diameter at the onset of the bevel. Instead of using an intermediate tube to provide the stepwise reduction in outer diameter from the outer tube to the tip of the needle, the reduction may also be established by the inner tube. Namely, the inner tube may have a tapered design which narrows down, for example in a stepwise manner or gradually, from the intermediate outer diameter to 120mm or below at the tip. Such a design may avoid the need for an intermediate tube while still providing sufficient axial stability and allowing sufficient visibility of the insertion site. In this respect, it is noted that the use of a tapered inner tube may not preclude the use of an intermediate tube.

Optionally, the outer tube is circumferentially tapered-down along a longitudinal direction from the outer tube outer diameter at its proximate end to the intermediate outer diameter. Instead of using an intermediate tube to provide the stepwise reduction in outer diameter from the outer tube to the tip of the needle, the reduction may also be established by the outer tube. Namely, the outer tube may have a tapered design which narrows down, for example in a stepwise manner or gradually, from the outer tube outer diameter to the intermediate outer diameter. Such a design may avoid the need for an intermediate tube while still providing sufficient axial stability and allowing sufficient visibility of the insertion site. In this respect, it is noted that the use of a tapered outer tube may not preclude the use of an intermediate tube.

Optionally, the needle further comprises:
- a shielding tube, wherein the concentric assembly is slidingly arranged in the shielding tube; and
- a telescoping mechanism arranged to extend the concentric assembly from and retract the concentric assembly into the shielding tube.

By providing a shielding tube, the concentric assembly, and in particular the tip of the inner tube, may be protected, for example when inserting the needle into the eye. This may avoid or reduce the chance of damage to the comparatively fragile tip of the inner tube. By providing a telescoping mechanism, the concentric assembly may be extended from the shielding tube once the needle is inserted into the eye.

Optionally, the telescoping mechanism comprises:
- a first body affixed to the shielding tube, for example by enveloping part of the shielding tube; and
- a second body affixed to the outer tube of the concentric assembly, for example by enveloping part of the outer tube, wherein the second body is slidingly arranged with respect to the first body to enable the concentric assembly to be extended from and retract into the shielding tube by relative movement between the second body and the first body.

By providing a telescoping mechanism in form of two bodies which are slidingly arranged with respect to each other, the telescoping mechanism may be easily actuated by moving the two bodies relative to each other. For example, the telescoping mechanism may be arranged at a proximate end of the needle which remains outside of the eye during surgery. The telescoping mechanism may thereby be actuated from outside of the eye, e.g., by hand or using a computer-controlled actuator.

Optionally, one of the first body and the second body is an outer body comprising a cavity and another one of the first body and the second body is an inner body slidingly arranged within the cavity of the outer body.

Optionally, the outer body comprises a gripping surface and wherein a part of the inner body is arranged to remain outside of the cavity to provide another gripping surface. Gripping surfaces may facilitate the actuation of the telescoping mechanism.

Optionally, the telescoping mechanism is arranged to:
- retract the tip of the inner tube by at least 0.1 mm, preferably by at least 0.5mm, into the shielding tube; and/or
- extend the tip of the inner tube by 3mm ± 1 mm from the shielding tube.

A retraction of at least 0.1mm may provide a reasonable degree of protection for the tip of the inner tube. An extension of at least 2mm, or preferably 3mm, from the shielding tube may prevent the shielding tube from obstructing the visibility of the insertion site, or at least limit the degree of obstruction.

A further aspect of the presently disclosed subject matter provides a needle system comprising the needle, wherein the needle system further comprises a flexible tubing which is connected to a proximate end of the outer tube of the concentric assembly, for example by a distal end of the flexible tubing being sleeved over the proximate end of the outer tube. The proximate end of the outer tube, and thereby the proximate end of the injection lumen, may remain outside of the eye during surgery. To enable a substance to be injected into the retina, the substance may be delivered to the proximate end of the injection lumen using a flexible tubing. By sleeving the flexible tubing over the outer tube, the use of additional connecting structures may be avoided, and the potential for air cavities, which may otherwise manifest themselves in such connecting structures, may be reduced. Air cavities may generally be undesirable in a needle and needle system since they may lead to inaccuracies in dosing.

Optionally, the flexible tubing comprises or is made out of a plastic, preferably a thermoplastic, more preferably a thermoplastic polymer such as polyimide.

Optionally, the flexible tubing is a braid reinforced tubing, for example by comprising a metal or fiber braiding. The braided tubing may resist kinking while remaining flexible, thereby facilitating easy manipulation of the needle during surgery.

Optionally, the needle system further comprises a syringe connector, wherein a proximate end of the flexible tubing is connected to a barb of the syringe connector, for example by being sleeved over the barb or internally fitted into the barb. The syringe connector may for example be a Luer connector of a Luer system, for example a female Luer connector. By sleeving the flexible tubing over the barb of the syringe connector or by internally fitting the flexible tubing into the barb, the use of additional connecting structures between the flexible tubing and the syringe connector may be avoided, and the potential for air cavities, which may otherwise manifest themselves in such connecting structures, may be reduced.

A further aspect of the presently disclosed subject matter provides a use of the needle for subretinal injection. For example, the needle as presently described may be used to subretinally inject a therapeutic agent below a retinal surface layer.

It will be appreciated by those skilled in the art that two or more of the above-mentioned embodiments, implementations, and/or aspects of the presently disclosed subject matter may be combined in any way deemed useful.

Modifications and variations of the needle, the needle system, and the uses of the needle or needle system, which correspond to the described modifications, variations, and optional aspects of another one of these entities and activities, may be carried out by a person skilled in the art on the basis of the present description.

### BRIEF DESCRIPTION OF THE DRAWINGS

These and other aspects of the presently disclosed subject matter are apparent from and will be elucidated with reference to the embodiments described hereinafter. In the drawings,
Fig. 1A shows a side view of a concentric assembly of a needle;
Fig. 1B shows a longitudinal cross-section of the concentric assembly;
Fig. 1C shows a transverse cross-section of the concentric assembly;
Fig. 2A shows a longitudinal cross-section of the concentric assembly and a shielding tube, wherein the concentric assembly is retracted into the shielding tube;
Fig. 2B shows a side view of the shielding tube when the concentric assembly is retracted into the shielding tube;
Fig. 2C shows a side view of the shielding tube when the concentric assembly is extended from the shielding tube;
Fig. 3 shows a longitudinal cross-section of a telescoping mechanism which comprises a first body affixed to the shielding tube and a second body affixed to an outer tube of the concentric assembly, wherein the second body is slidingly arranged with respect to the first body to enable the concentric assembly to be extended from and retract into the shielding tube by relative movement between both bodies;
Fig. 4 shows a longitudinal cross-section of a proximate end of the outer tube and a flexible tubing which is sleeved over the outer tube; and
Fig. 5 shows a proximate end of the flexible tubing, wherein the flexible tubing is sleeved over a barb of a syringe connector.

It should be noted that items which have the same reference numbers in different figures, have the same structural features and the same functions, or are the same signals. Where the function and/or structure of such an item has been explained, there is no necessity for repeated explanation thereof in the detailed description.

### List of reference numerals

The following list of references and abbreviations is provided for facilitating the interpretation of the drawings and shall not be construed as limiting the claims.
- 100: concentric assembly
- 110: inner tube
- 112: inner tube outer diameter
- 114: inner tube protrusion length
- 116: beveled tip
- 120: intermediate tube
- 122: intermediate tube outer diameter
- 124: intermediate tube protrusion length
- 130: outer tube
- 132: outer tube outer diameter
- 140: lumen
- 150: cross-sectional plane

- 200: shielding tube
- 210: first body
- 212: cavity
- 220: second body
- 230: retraction length
- 240: telescopic extension length
- 250: sliding movement

- 300: flexible tubing
- 310: syringe connector
- 320: barb

### DESCRIPTION OF EMBODIMENTS

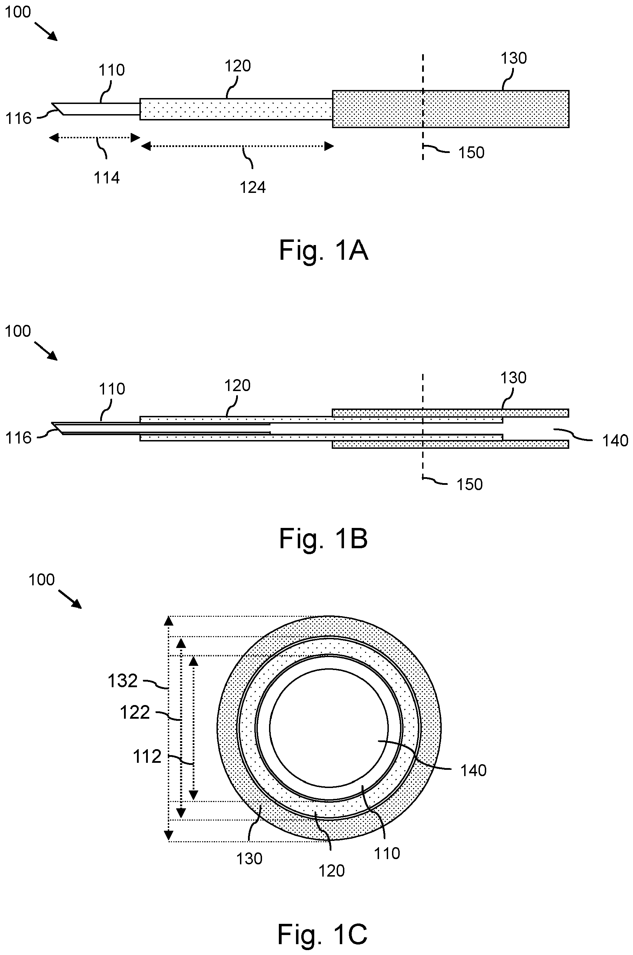

**Fig. 1A** shows a side view of a distal section of a concentric assembly 100 of a needle. The distal section of the concentric assembly 100 may enter a patient's eye during surgery. The needle may further comprise a proximate section (not shown in Fig. 1A) which may remain outside of the eye during surgery. For example, the proximate section of the needle may comprise a handle to enable the needle to be manipulated, e.g., by human hand or by a surgical robotic system or by any other mechanism designed to support manipulation of the needle. In this respect, it is noted that the terms 'distal' and 'proximate' may assume an operator of the needle as a point of reference, meaning that a distal section or end may be located away from the operator and typically nearest to a surgical target, while a proximate section or end may be located towards the operator and typically farthest from the surgical target.

The concentric assembly 100, which may extend in longitudinal direction beyond the length shown in Fig. 1A, may comprise an outer tube 130 and an inner tube 110 concentrically arranged within the outer tube 130. The inner tube 110 may comprise a tip 116 for piercing a retinal surface layer, with the tip 116 being arranged at a distal end of the inner tube 110. The concentric assembly 100 may be arranged to circumferentially taper-down along a longitudinal direction towards the tip 116 from the outer tube 130 to the inner tube 110, meaning that the outer diameter of the concentric assembly 100 may be reduced from an outer diameter of the outer tube 130 via an intermediate outer diameter to an outer diameter of the inner tube 110. In the example of Fig. 1A and following, this circumferential reduction is provided by the concentric assembly 100 comprising an intermediate tube 120 concentrically arranged between the inner tube 110 and the outer tube 130. While the outer diameter of the inner tube 110 and the outer diameter of the outer tube 130 may be substantially constant in the distal section of the concentric assembly 100 or along the length of the concentric assembly 100, the intermediate tube 120 may provide an intermediate outer diameter and thereby a stepwise reduction in outer diameter from the outer diameter of the outer tube 130 to the outer diameter of the inner tube 110. It will be appreciated that in addition or alternatively to the use of an intermediate tube 120, the outer tube 130 and/or the inner tube 110 may be tapered so that a respective tube comprises an outer diameter which varies over a range which includes the intermediate outer diameter.

Fig. 1A further shows the intermediate tube 120 extending axially from a distal end of the outer tube 130 and the inner tube 110 extending axially from a distal end of the intermediate tube 120. For example, the inner tube 110 may extend axially from the distal end of the intermediate tube 120 by a length 114 of 1mm or less, or by 750µm or less, or by 500µm ± 100µm, or by 500µm, while the intermediate tube 120 may extend axially from the distal end of the outer tube 130 by a length 124 of 1mm to 3mm, or by a length of 2mm ± 500µm, or by 2mm ± 250µm, or by 2mm. It is noted that by extending axially outward from the intermediate tube 120, the inner tube 110 also extends axially outward from the outer tube, e.g., by a greater length. It will be further appreciated that Figs. 1A and following are not drawn to scale and that any dimensions shown in the figures may not be representative of dimensions as described.

Fig. 1A shows the tip 116 of the inner tube 110 as being beveled. The bevel angle may for example be 45° ± 10°, or 45° ± 5°, or 45° ± 2.5°, or 45°.

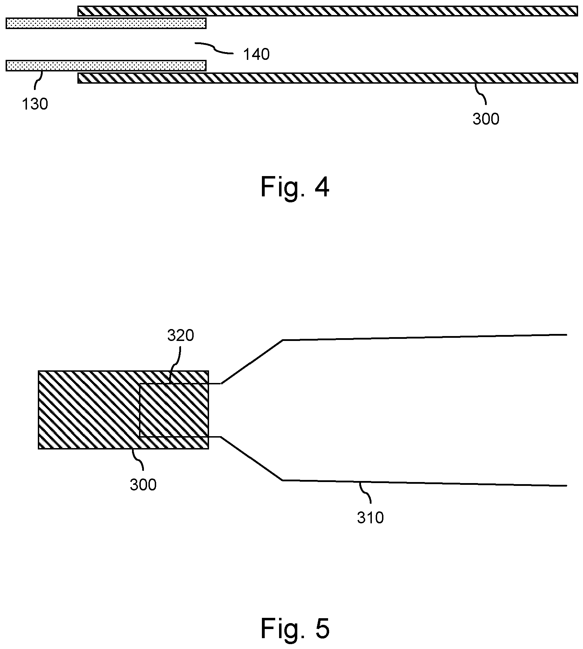

**Fig. 1B** shows a longitudinal cross-section of the concentric assembly 100 which shows that the concentric assembly 100 may form a lumen 140 for injecting a substance. Specifically, the lumen 140 may be formed by the interior walls of the respective tubes 110, 120, 130. The lumen 140 may comprise a first opening at the tip 116 of the inner tube 110, with the first opening being elsewhere also referred to as an exit aperture, and a second opening at a proximate section of the needle (later shown in Fig. 4). The substance may be supplied via the second opening and then delivered through the lumen 140 within the needle to the tip 116, where the substance may be expelled from the first opening and thereby injected into retinal tissue. Fig. 1B further shows that the intermediate tube 120 may not extend internally within the outer tube 130 along the full length of the outer tube 130, while the inner tube 110 may not extend internally within the intermediate tube 120 along the full length of the intermediate tube 120. It will be appreciated, however, that the interior length of the intermediate tube 120 and the interior length of the inner tube 110 as shown in Fig. 1B may be merely exemplary and may differ in practice. For example, the inner tube 110 may extend internally along the full length of the intermediate tube 120 and the intermediate tube 120 may extend internally along the full length of the outer tube 130. Another example is that the inner tube 110 may extend internally within the intermediate tube 120 by the length, or at least by the length, by which the inner tube 110 axially extends from the intermediate tube 120 (see reference numeral 114 in Fig. 1A), and the intermediate tube 120 may extend internally within the outer tube 130 by the length, or at least by the length, by which the intermediate tube 120 axially extends from the outer tube 130 (see reference numeral 124 in Fig. 1A). Another example is that the inner tube 110 may extend internally within the intermediate tube 120 by approximately twice the diameter, or by at least twice the diameter, of the inner tube 110 or of the intermediate tube 120, and the intermediate tube 120 may extend internally within the outer tube 130 by approximately twice the diameter, or by at least twice the diameter, of the intermediate tube 120 or of the outer tube 130.

**Fig. 1C** shows a transverse cross-section of the concentric assembly of Figs. 1A and 1B, with the cross-sectional plane of Fig. 1C being represented by a dashed line 150 in Figs. 1A and 1B. It can be seen that the outer tube 130 comprises an outer tube outer diameter 132, the intermediate tube 120 comprises an intermediate tube outer diameter 122, and the inner tube 110 comprises an inner tube outer diameter 112. In some examples, these diameters may be substantially constant along the lengths of the respective tubes, while in other examples, one or more of the outer diameters may vary along the length of a respective tube. As can be further seen in Fig. 1C, the outer diameter 112 of the inner tube 110 may be selected to substantially match an inner diameter of the intermediate tube 120, and the outer diameter 122 of the intermediate tube 120 may be selected to substantially match an inner diameter of the outer tube 130. This may facilitate attachment of the inner tube 110 to an inside of the intermediate tube 120 and attachment of the intermediate tube 120 to an inside of the outer tube 130. For example, the inner tube 110 may be affixed with an adhesive, such as glue, to the inside of the intermediate tube 120 and the intermediate tube 10 may be affixed with an adhesive, such as glue, to the inside of the outer tube 130. Other examples to attach respective tubes to each other may be interference fitting or press fitting of respective tubes, heat shrinking, ultrasonic welding, etc. With continued reference to outer diameter 112 of the inner tube 110, the outer diameter 112 may be selected to be 120µm or less at an onset of the bevel, or 110µm or less, or 100µm or less, or 80µm ± 10µm, or 80µm. With continued reference to outer diameter 122 of the intermediate tube 120, the outer diameter 122 may be between 140µm and 180µm, for example 160µm ± 10µm or 160µm.

The inner tube 110 of the concentric assembly 100 may comprise or may be made of a material which is at least partially translucent to OCT. For example, the inner tube 110 may comprise or may be made out of a translucent plastic, for example a translucent thermoplastic. In a specific example, the inner tube 110 may comprise or may be made out of a translucent polymer such as polyimide. Materials which may be considered to be at least partially translucent to visible light may provide a sufficient degree of translucency to OCT. Polyimide may be naturally translucent to visible light. The intermediate tube 120 may in some examples also comprise or may be made of a material which is at least partially translucent to OCT. For example, the intermediate tube 120 may comprise or may be made out of a translucent plastic, for example a translucent thermoplastic. In a specific example, the intermediate tube 120 may comprise or may be made out of a translucent polymer such as polyimide. In a specific example, both the inner tube 110 and the intermediate tube 120 may comprise or may be made out of the same material, for example polyimide. In other examples, the intermediate tube 120 may comprise or may be made out of a different material, for example a non-translucent material such as metal. An example of a metal may be stainless steel, e.g., AISI 304 or AISI 316. The outer tube 120 may comprise or may be made out of metal, for example stainless steel, e.g., AISI 304 or AISI 316, or any material known to provide sufficient structural strength in a needle application.

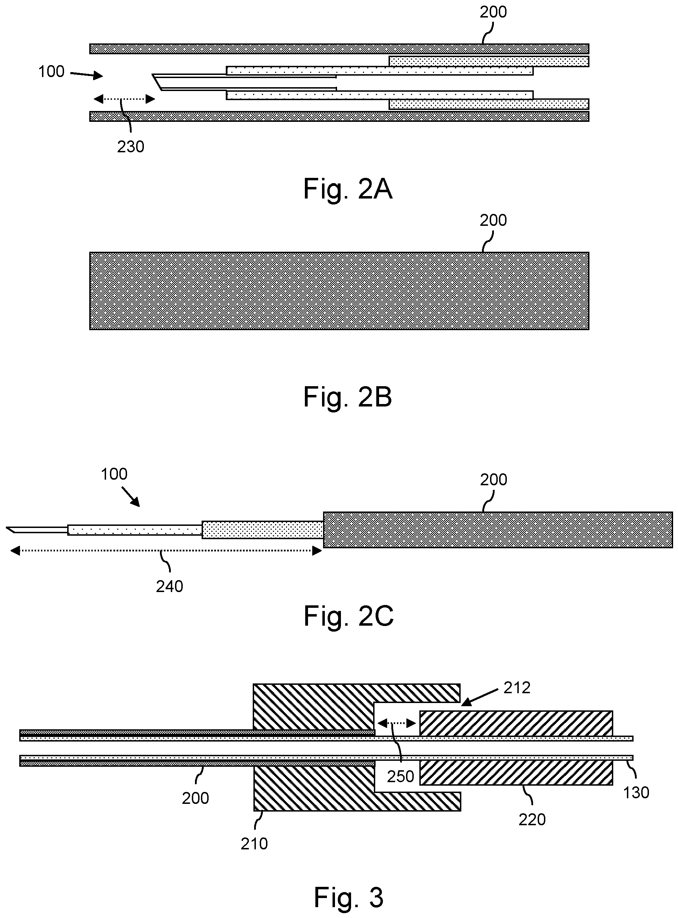

**Fig. 2A** shows a longitudinal cross-section of the distal end of the concentric assembly 100 and a shielding tube 200. The concentric assembly 100 may be slidingly arranged in the shielding tube 200. As also elucidated with reference to Fig. 3, and not shown in Fig. 2A, the needle may further comprise a telescoping mechanism arranged to extend the distal end of the concentric assembly 100 from and retract the distal end of the concentric assembly into the shielding tube 200. Fig. 2A shows the concentric assembly 100 in a retracted state. **Fig. 2B** shows a side view of the shielding tube 200 when the concentric assembly is retracted into the shielding tube. For example, and with continued reference to Fig. 2A, in such a retracted state, the tip of the concentric assembly 100 may be retracted by a length 230 of at least 0.1 mm from a distal opening of the shielding tube 200. In another example, the tip of the concentric assembly 100 may be retracted by a length 230 of at least 1x, 1.5x, or 2x the inner diameter of the shielding tube. For example, the tip of the concentric assembly 100 may be retracted by a length 230 of at least 0.5mm into the shielding tube 120. The shielding tube may for example comprise or may be made out of metal, for example stainless steel, e.g., AISI 304 or AISI 316. The shielding tube 230 may shield a distal end of the concentric assembly as shown in Figs. 2A and 2B but may not need to shield a proximate end of the concentric assembly, for example to allow the lumen formed by the concentric assembly to be connected to flexible tubing.

**Fig. 2C** shows a side view of the shielding tube 200 when the concentric assembly 100 is extended by the telescoping mechanism from the shielding tube 200. For example, when extended, the tip of the inner tube may be extended by a length 240 of 3mm +/- 1mm, or by a length 240 of 3mm, from the shielding tube 200.

**Fig. 3** shows a longitudinal cross-section of an example of a telescoping mechanism. The telescoping mechanism as shown in Fig. 3 may comprise a first body 210 affixed to the shielding tube 200, for example by enveloping part of the shielding tube 200, and a second body affixed to the outer tube 130 of the concentric assembly, for example by enveloping part of the outer tube 130. The telescoping mechanism may be located at a proximate section of the needle which may remain outside of the eye during surgery. Thereby, the telescoping mechanism may be actuatable from outside of the eye, e.g., by human hand or by a surgical robotic system. The second body 220 may be slidingly arranged with respect to the first body 210 to enable the concentric assembly to be extended from and retract into the shielding tube 200 by relative movement between both bodies 210, 220. The sliding arrangement of both bodies 210, 220 may for example be established as follows: the relative movement of the outer tube 130 and the shielding tube 200 may be constrained to sliding movement 250 due to their nested arrangement, and as a result, the bodies 210, 220, owning to their affixation to the respective tubes, may also be constrained to sliding movement. Another factor which may establish the sliding arrangement of the bodies 210, 220 may be a complementary shape of both bodies 210, 220. For example, the first body 210 may comprise a cavity 212 and the second body 220 may be slidingly arranged within the cavity 212 of the first body 210. In another example, not shown in Fig. 3, the second body may comprise a cavity and the first body may be slidingly arranged within the cavity of the second body. One or both bodies 210, 220 may be arranged to be gripped at their respective surfaces. A surface suitable for gripping may elsewhere also be referred to as a gripping surface. By gripping the first body 210 and separately gripping the second body 220, both bodies 210, 220 may be slidingly moved with respect to each other to actuate the telescoping mechanism. In some examples, one of the bodies 210, 220 may serve as a handle of the needle. In another example, the first body 210 and the second body 220 may each or jointly serve as the handle. In another example, not shown in Fig. 3, the sliding arrangement of both bodies 210 and 220 may prevent or limit their mutual rotation. For example, the bodies 210, 220 may have complementary yet rotationally asymmetric shapes that restrict their mutual rotation. For example, rotational asymmetry may be provided by one of the bodies having a longitudinal bevel and the other body having a corresponding longitudinal groove or notch. Additionally, or alternatively, one or more alignment marks may be provided to ensure both bodies are rotationally aligned. For example, each body may have an alignment mark, and both alignment marks may be mutually aligned through mutual rotation of both bodies 210, 220 when or before using the needle during surgery.

It will be appreciated that the telescoping mechanism shown in Fig. 3 is an example and that any other, for example known type of telescoping mechanism may be used instead. The needle may also omit a telescopic mechanism and may instead use a different mechanism to temporarily shield the distal end of the concentric assembly, for example when entering the eye, or omit such shielding entirely.

**Fig. 4** shows a longitudinal cross-section of a proximate end of the outer tube 130 of the concentric assembly. The proximate end of the outer tube 130 may remain outside of the eye during surgery while the distal end of the outer tube 130 may be inserted into the eye, for example via a trocar. When the needle is equipped with a telescoping mechanism as shown in Fig. 3, the proximate end of the outer tube 130 may be located beyond the telescoping mechanism towards the proximate end of the needle. Fig. 4 shows a flexible tubing 300 which is sleeved over the outer tube 130. More specifically, the flexible tubing 300 may be connected to the proximate end of the outer tube by a distal end of the flexible tubing 300 being sleeved over the proximate end of the outer tube 130. The flexible tubing 300 may be used to supply a substance into the lumen 140 of the needle. The needle, which may comprise a concentric assembly and optionally a shielding tube, a telescoping mechanism, and other optional components, may together with the flexible tubing 300 form a needle system. The interior of the flexible tubing 300 may, together with the lumen 140 formed by the concentric assembly, form a lumen of the needle system. The flexible tubing 300 may comprise or may be made out of a plastic, such as a thermoplastic, or specifically a thermoplastic polymer such as polyimide. The flexible tubing 300 may be reinforced by braiding. For example, the flexible tubing 300 may comprise a metal or fiber braiding. In a specific example, the flexible tubing 300 may be a metal or fiber braided polyimide tubing.

**Fig. 5** shows a proximate end of the flexible tubing 300. The proximate end of the flexible tubing 300 may comprise an opening through which a substance may be supplied into the flexible tubing and subsequently into the lumen formed by the concentric assembly of the needle. For example, the substance may be supplied by a syringe. For that purpose, the flexible tubing 300 may be connected to a syringe connector 310, such as a Luer connector. The syringe connector 310 may comprise a barb 320. The barb 320 may comprise an opening through which the substance may be ejected. As shown in Fig. 5, the flexible tubing 300 may be sleeved over (part of) the barb 320. Alternatively, the flexible tubing 300 may be internally fitted into the barb 320.

Embodiments of the presently disclosed subject matter may be based on one or more of the following insights and/or may have one or more of the following advantages. In the field of subretinal injections, a problem may be a lack of control to ensure dependable subretinal injection with current designs of SRI needles. This lack of control may result in an uncertainty if a bleb will be formed, e.g., due to backflow of the substance into the vitreous cavity, and uncertainty in whether the substance is injected at the correct location, e.g., at the correct injection depth. The backflow, which may also be referred to as reflux, may affect the surgical efficacy and/or performance and may lead to an inflammation reaction of the retina, potentially requiring follow-up treatments or leading to blindness. Embodiments of the presently disclosed subject matter may provide a needle tip design which may allow for substance delivery at a suitable subretinal location while avoiding or minimizing reflux of the substance into the vitreous cavity. Embodiments of the presently disclosed subject matter may further provide a tubing design and/or lumen design which may allow highly responsive injection flow control and which may minimize so-called dead volume.

A specific embodiment of presently disclosed subject matter may provide a micro-SRI needle with a plastic tip comprising an outer diameter of <100µm. The tip may be optimized for retinal penetration control and positioning in the eye anatomy layers. The lumen and tubing of the needle and needle system incorporating the needle may be optimized for injection control and low dead volume. The micro-SRI needle may comprise an outer body which may be held manually or robotically or by any other mechanism designed to support manipulation of the needle. To the outer body, a shielding tube may be fixated that may shield the micro-SRI needle when retracted. Inside and extending from the proximal end of the outer body, an inner sliding body may be provided which may comprise a concentric assembly. This concentric assembly may comprise a number of concentrically arranged tubes, such as an outer metal tube which may function as a needle carrier and which may be directly, or via a stiffening tube, attached to the inner sliding body. At a distal end of the outer metal tube, an intermediate polyimide tube with an outer diameter of 140µm lumen may be provided to strengthen and carry a further inner tube with an outer diameter of <100µm that extends 500µm from the intermediate tube. The tip may comprise a 45° bevel. The inner sliding body may have a sliding capacity with respect to the outer fixed body of about 4 mm, for example allowing the tip to be retracted by about 1mm into the shielding tube or allowing the tip to be extended by about 3mm from the shielding tube.

The tubing design and/or lumen design, ranging from a distal end of the needle, e.g., from its tip, towards a syringe, may comprise a number of concentric parts, for example the aforementioned concentric assembly of tubes and flexible tubing. Transitions between parts may be such that potential volumes for air cavities may be minimized. For example, the inner tube may have an outer diameter which may precisely fit into the intermediate tube, and the intermediate tube may have an outer diameter which may precisely fit into the outer metal tube. At a proximate end of the outer tube, a braided flexible polyimide tube may be placed over the outer tube, which braided flexible polyimide tube may elsewhere also be referred to as flexible tubing. The length of the flexible tubing may be selected as desired, for example to position the syringe either close or at a distance from the needle. A proximate end of the flexible tubing may be fitted inside of a blunt needle or in any other manner interface with a syringe. Each respective transition between parts may be fixated using an adhesive such as glue. Since the transitions between parts may be relatively small, air cavities may be kept to a minimum. The material of the flexible tubing may be selected for being stiff against expansion, kink resistant, while maintaining a high amount of flexibility for motion. The relatively small inner diameter of the flexible tubing, for example precisely fitting the outer diameter of the outer tube, may contribute to the small dead volume (also referred to as ullage) of the needle system. For example, in a specific example of the needle system, the dead volume may be 63 µL when the needle system has a length of 60cm. The inner diameter of the flexible tubing may be selected to be as small as possible while still allowing the substance to be delivered to the tip of the needle using limited pressure, e.g., <10PSI.

While the presently disclosed needle may be used for the subretinal injection of a substance, the needle is not limited to such uses. Rather, subretinal aspiration uses are also envisioned, as well as non-subretinal applications, such as intravitreal, intracameral, or subcutaneous injections. The substance to be injected may be a fluid. For example, the fluid may be or comprise a therapeutic agent, which may encompass a wide range of liquid formulations including solutions, suspensions, emulsions, or colloids. The therapeutic agent may contain active pharmaceutical ingredients such as drugs, biological agents including proteins, peptides, nucleic acids, or cells, as well as excipients or additives like stabilizers, preservatives, or buffers. Additionally, the fluid may be designed for various therapeutic purposes such as analgesic, antibiotic, anti-inflammatory, or chemotherapeutic treatments.

It should be noted that the above-mentioned embodiments illustrate rather than limit the presently disclosed subject matter, and that those skilled in the art will be able to design many alternative embodiments without departing from the scope of the appended claims.

In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of" when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The mere fact that certain measures are recited in mutually different dependent claims does not indicate that a combination of these measures cannot be used to advantage.

## Claims

1. A needle for subretinal injection, the needle comprising a concentric assembly (100) forming a lumen (140) for a substance to be injected below a retinal surface layer, the concentric assembly comprising:
- an outer tube (130) comprising an outer tube outer diameter (132); and
- an inner tube (110) comprising a tip (116) for piercing the retinal surface layer, wherein the tip is at a distal end of the inner tube, wherein the inner tube extends axially from a distal end of the outer tube, wherein the inner tube is at least partially translucent to optical coherence tomography (OCT), wherein the tip is beveled, wherein the inner tube has an inner tube outer diameter (112) of 120µm or less at an onset of the bevel; and
wherein the concentric assembly is arranged to circumferentially taper-down along a longitudinal direction towards the tip from the outer tube outer diameter, via at least one intermediate outer diameter (122), to the inner tube outer diameter.

2. The needle according to claim 1, wherein the inner tube outer diameter (112) is 110µm or less, preferably 100µm or less, more preferably 80µm ± 10µm.

3. The needle according to claim 1 or 2, wherein the bevel of the tip (116) is beveled at an angle of 45° ± 10°, preferably 45° ± 5°, more preferably 45° ± 2.5°.

4. The needle according to any one of claims 1 to 3, wherein the inner tube (110) comprises or is made out of a translucent plastic, preferably a translucent thermoplastic, more preferably a translucent polymer such as polyimide.

5. The needle according to any one of claims 1 to 4, wherein the concentric assembly (100) further comprises an intermediate tube (120) concentrically arranged between the inner tube (110) and the outer tube (130), the intermediate tube comprising the intermediate outer diameter (122), wherein the intermediate tube extends axially from the distal end of the outer tube and the inner tube extends axially from a distal end of the intermediate tube.

6. The needle according to claim 5, wherein:
- the inner tube outer diameter (112) is selected to substantially match an intermediate inner diameter of the intermediate tube; and/or
- an intermediate tube outer diameter (122) of the intermediate tube is selected to substantially match an outer tube inner diameter of the outer tube.

7. The needle according to any one of claims 1 to 6, wherein the inner tube (110) is circumferentially tapered-down along a longitudinal direction from or via the intermediate outer diameter to the inner tube outer diameter (112) at the onset of the bevel.

8. The needle according to any one of claims 1 to 7, wherein the outer tube (130) is circumferentially tapered-down along a longitudinal direction from the outer tube outer diameter (132) at its proximate end to the intermediate outer diameter.

9. The needle according to any one of claims 1 to 8, further comprising:
- a shielding tube (200), wherein the concentric assembly is slidingly arranged in the shielding tube; and
- a telescoping mechanism (210, 220) arranged to extend the concentric assembly from and retract the concentric assembly into the shielding tube.

10. The needle according to claim 9, wherein the telescoping mechanism comprises:
- a first body (210) affixed to the shielding tube (200), for example by enveloping part of the shielding tube; and
- a second body (220) affixed to the outer tube (130) of the concentric assembly (100), for example by enveloping part of the outer tube, wherein the second body is slidingly arranged with respect to the first body to enable the concentric assembly to be extended from and retract into the shielding tube by relative movement (250) between the second body and the first body.

11. The needle according to claim 10, wherein one of the first body (210) and the second body (220) is an outer body comprising a cavity and another one of the first body and the second body is an inner body slidingly arranged within the cavity of the outer body.

12. A needle system comprising the needle according to any one of claims 1 to 11, wherein the needle system further comprises a flexible tubing (300) which is connected to a proximate end of the outer tube (130) of the concentric assembly (100), for example by a distal end of the flexible tubing being sleeved over the proximate end of the outer tube.

13. The needle system according to claim 12, wherein the flexible tubing (300) comprises or is made out of a plastic, preferably a thermoplastic, more preferably a thermoplastic polymer such as polyimide, wherein the flexible tubing is a braid reinforced tubing, for example by comprising a metal or fiber braiding.

14. The needle system according to claim 12 or 13, further comprising a syringe connector (310), wherein a proximate end of the flexible tubing (300) is connected to a barb (320) of the syringe connector, for example by being sleeved over the barb or internally fitted into the barb.

15. Use of a needle as defined in any one of claims 1 to 14 for subretinal injection.
